Europäisches Patentamt

European Patent Office

Office européen des brevets

(19)

(11) Publication number: **0 025 704**
**B1**

(12) **EUROPEAN PATENT SPECIFICATION**

(45) Date of publication of patent specification: **19.10.83**

(51) Int. Cl.³: **A 61 M 25/00, A 61 M 1/03**

(21) Application number: **80303213.5**

(22) Date of filing: **12.09.80**

(54) **A double lumen cannula.**

(30) Priority: **17.09.79 US 76101**

(43) Date of publication of application:
**25.03.81 Bulletin 81/12**

(45) Publication of the grant of the patent:
**19.10.83 Bulletin 83/42**

(84) Designated Contracting States:
**BE DE FR GB IT NL**

(56) References cited:
**EP - A - 0 000 041**
**DE - A - 3 006 291**
**GB - A - 1 284 537**
**GB - A - 2 017 499**
**US - A - 4 106 506**

(73) Proprietor: **SORENSON RESEARCH CO. INC.**
**4387 Atherton Drive**
**Salt Lake City Utah 84107 (US)**

(72) Inventor: **Uldall, Peter Robert**
**11 Birchwood Avenue**
**Willowdale M2L 1M2 Ontario (CA)**

(74) Representative: **Walters, Frederick James et al,**
**Urquhart-Dykes & Lord 47 Marylebone Lane**
**London W1M 6DL (GB)**

Courier Press, Leamington Spa, England

A double lumen cannula.

Technical Field

This invention relates to a double lumen cannula, and more particularly to a cannula which can be used for hemodialysis and can be inserted in semi-permanent manner into the subclavian vein of the patient. For regular hemodialysis, permanent vascular access is normally provided by means of a surgically constructed arteriovenous fistula, created if possible in advance of need.

Background Art

The conventional method of conducting hemodialysis on a patient is to introduce into an arterialised vein, normally a lower arm vein, one or two blood flow needles, to remove blood from the patient to the exterior hemodialysis machine and to return the treated blood to the patient at substantially the same location. At least one puncture of the vein needs to be made for such catheter insertion every time the patient undergoes a hemodialysis treatment. Whilst in common practice two separate needle devices are used, one for blood overflow and the other for blood return, there are several proposals for the use of a single catheter provided with co-axially arranged lumen, so as to reduce to one the number of vein punctures required in each hemodialysis treatment. Examples of co-axially arranged metal catheters for use in conventional hemodialysis, in a limb of the patient, are to be found in U.S. Patents Nos. 4,037,599; 4,073,297; 4,134,402, and 4,096,860. All of these examples of prior art show a rigid, metal needle-type catheter for temporary use during the actual hemodialysis or transfusion etc. operation on the patient, and removal once the operation is completed. In each case the rigid inner tube protrudes beyond the outer tube. None is flexible or suitable for insertion into the subclavian vein.

Although the arterio-venous fistula is the standard and accepted method for permanent vascular access, unfortunately, some patients experience end stage renal failure without warning, and established fistulae may fail unexpectedly. With the growth of large programs for long-term peritoneal dialysis, an increasing number of patients require to be transferred at short notice to hemodialysis because of peritonitis. Such patients do not usually have arterio-venous fistulae constructed in advance, since many of them will never need them. Patients on long-term peritoneal dialysis may also need short-term hemodialysis while they undergo abdominal surgery. Transplant recipients whose arterio-venous fistulae have thrombosed may develop acute renal failure. For all these categories of patients, the silastic teflon shunt, though immediately usable, wastes blood vessels and may not be feasible in patients whose access sites have already been used. Temporary peritoneal dialysis is not always a suitable alternative.

There is thus a need for a simple, immediately usable vascular access method which does not destroy blood vessels, and does not limit the patient's mobility. Temporary vascular access for hemodialysis can be obtained with a femoral cannula introduced by the Seldinger technique, normally inserted in the femoral vein.

With such prior art device, the patient must be prepared and the catheter applied carefully, by medically trained personnel, prior to every hemodialysis use. For patients with absence of renal function, this can be several times per week. The preparation and application is time-consuming and difficult to perform on an emergency basis. The cannula cannot be left in situ after dialysis if the patient is to remain mobile. Moreover, repeated insertions lead to the build-up of scar tissue at the access sites of the patient. Some means of readily usable, semi-permanent vascular access would be preferable.

Summary of the Invention

In the present invention, there is provided an indwelling subclavian cannula which is particularly suitable for temporary hemodialysis. When not in use, it remains in situ without restricting the mobility or activities of the patient to any significant extent. The invention provides a double lumen cannula, with the lumens located co-axially, so that only one insertion into the vein of the patient is required. Both of the lumens are elongated and flexible so as to assume the disposition of the subclavian vein. The inner lumen is removable from within the outer lumen, without removing the cannula as a whole from the subclavian vein of the patient, thereby providing for replacement of the inner lumen and cleaning and access of the apparatus as a whole.

Thus according to the present invention, there is provided a double luman cannula suitable for insertion into a large vein such as the subclavian vein of the patient for blood removal therefrom and blood return thereto, said cannula comprising:

outer and inner tubular members (12 and 14) and characterised in that the outer tubular member (12) is elongated and flexible with a smooth outer surface and comprises a main elongated portion (24) of first cross-section, an integral convergent section (26), an integral distal end portion (28) adjoining the convergent section and having a second cross-section which is smaller than the first cross-section, a plurality of blood flow apertures (34) spaced apart from one another in the side wall of the main portion adjacent the convergent section (26), and at least one blood flow aperture (30, 32) in the distal end portion (28), and further characterised in that the inner tubular member (14) is in situ within the outer tubular member (12) and is elongated and flexible with a smaller cross-section than the first cross-section and is releasably and withdrawably disposed within the outer member (12) with its distal end within the outer lumen and in sealing end contact with the convergent section (26) of the outer member so as to define with the outer member (12) two concentrically arranged discrete fluid flow lumens, an inner lumen being constituted by the inner member (14) and the distal end portion (28) of the outer member (12) and an outer lumen constituted by the main portion (24) and the convergent section (26) of the outer member (12).

The provision of two blood pathways for simultaneous extraction of blood from and return of blood to the patient obviates the need for use of a single needle machine to provide an alternating blood flow.

Brief Reference to the Drawings

Figure 1 is a perspective view of a specific preferred embodiment of the present invention;

Figure 2 is a view similar to Figure 1, on a larger scale, and omitting the middle part of cannula;

Figure 3 is a cross sectional in detail of the end of the cannula, with the inner lumen withdrawn;

Figure 4 is a view similar to Figure 3, but with the inner lumen in sealing engagement and contact with the outer lumen;

Figure 5 is a diagrammatic cross sectional view similar to Fig. 4 showing in schematic representation the path taken by blood during hemodialysis with the cannula in place.

Figure 6 is a diagrammatic view showing the cannula of the present invention in place in the subclavian vein of a patient.

Description of the Preferred Embodiments

The cannula according to the present invention is preferably constructed of flexible, radio-opaque tetrafluoroethylene polymer, having known biocompatibility for residence in the patient's body over extended periods of time, having smooth outer surfaces, and having the necessary degree of flexibility to assume the configuration of the subclavian vein and superior vena cava without kinking or buckling or otherwise constricting the flow of fluid therethrough. The length of the outer member should be such that, after proper insertion, the tip lies at the junction of the superior vena cava and right atrium of the patient, whilst extending along the subclavian vein to the exterior of the patient's body. It should not extend past the right atrium, or there is a risk that it may enter the heart. The diameter of the cannula must be small anough so that it does not damage the vein, and should be substantially smaller than the size of the subclavian vein itself. It should nevertheless be large enough to provide two pathways, to give a flow through each pathway of approximately 200 mls per minute, of blood. It must not, of course, have any external projections or the like which might tear the vein.

In addition, the preferred embodiment of the invention has a tapered tip at its distal, to facilitate its insertion, e.g. by the Seldinger technique.

The provision of an inner lumen which is removable from the outer lumen is a significant feature in the device of the present invention. In this arrangement, the inner lumen can be removed from the outer lumen to facilitate initial introduction of the cannula into the subclavian vein. The cannula is best inserted by means of the known Seldinger technique, in which the vein is punctured with a Seldinger needle, having a cuff, and then the needle is removed, leaving the cuff in place. The guidewire is inserted into the vein, through the cuff, the cuff then being removed. Subsequently, the cannula according to the present invention can be inserted over the guidewire, and can at this time contain an obturator, of stiffer but flexible material, so as to ensure that the outer lumen does not buckle or kink during insertion. Once it is safely in place, the obturator can be removed, and the inner lumen can then be inserted. The desirable use of an obturator in this manner is made possible by the feature of removability of the inner lumen.

Moreover, in the situation where the cannula is left inserted in the subclavian vein for two to three weeks, and the cannula is used 3 times per week or more for hemodialysis, it is necessary to ensure that the cannula does not become blocked with blood clots. This is normally done by means of heparin. With the arrangement of the present invention, the inner lumen can be removed, for more thorough cleaning and clot removal from both the inner lumen and the outer lumen. If desired, a specific declotting catheter can be inserted into the outer lumen, once the inner lumen has been removed therefrom.

Moreover, if the inner lumen breaks or cracks, due to it being thinner and weaker than the outer lumen, it can easily be replaced, even while dialysis is being conducted, and does not necessitate the removal and re-insertion of an entire cannula, into the patient. A supply of spare inner lumens can be provided, along with each cannula according to the invention, to safeguard against such emergencies.

The end of the cannula remote from its distal end is normally provided with a medical grade silicone rubber extension, to the end of which is sealingly secured a junction piece. The inner lumen passes through the silicone rubber extension and out through one arm of the junction pieces whilst the other arm of the junction piece is connected to the outer lumen. Then, the junction pieces can be connected to the respective dialysis machine connections. The junction piece suitably has a straight arm, through which the inner lumen passes and a second arm extending at an oblique angle in communication with the outer lumen. It is preferred to adjust the length of the outer lumen so that, when properly inserted, the end of the silastic extension is flush with the patient's skin, and extends outwardly therefrom. The silastic extension provides a means for closing the cannula by applying a clamp thereto. When not in use, the junction ends are suitably closed by means of injection caps with Luer locks. The cannula can be anchored in place by means of a sterile adhesive transparent dressing. One dressing is placed below and under the silastic extension and reflected back, adhesive side up. The second is placed above and overlapping the first, so that the silastic extension is enclosed between two layers of sterile dressing. Thus it is not necessary to suture the cannula in place, and the entre site is protected from contamination. After routine chest x-rays to check the cannula position, it is possible to start hemodialysis immediately. At the conclusion of the procedure, the cannula is flushed with heparinized saline, and both the junction pieces are sealed with injection caps with Luer locks. This allows injection of heparin periodically to maintain potency within both lumens of the cannula. Patients can safely leave the medical treatment facility with the cannula in situ, and the patient can inject the heparin himself when required. It is preferred to maintain the silastic segment closed by a sliding or spring clamp, as a further protection against blood loss or air embolus resulting from a disconnected cap, at all times when the subclavian cannula is not in use for dialysis or heparin injection.

For the average sized, adult patient, the outer member of the cannula should have a length of from about 18 to about 22 cms, for ensuring proper insertion. The combined length of the convergent section and distal end portion is suitably 3—4 cm.

It is also preferred to make both the inner and outer members of the cannula of the invention from a radio-opaque material. Then if necessary the correct installation and positioning of the cannula can be checked periodically.

Detailed Description of the Specific
Preferred Embodiment

Referring now to the drawings, a cannula 10 according to the present invention includes a tubular outer member or lumen 12, an insertable and withdrawable inner tubular member or lumen 14, a silicone rubber extension 16 sealingly attached to one end of the outer lumen 12 and a junction piece 18 having two arms 20, 22, the body of the junction piece 18 being attached to the other end of the silicone rubber extension 16, in sealing engagement therewith.

The outer lumen 12 has a main tubular elongated portion 24, an integral convergent section 26 and an integral distal end portion 28 adjoining the convergent section 26. The distal end portion is provided with a tapered end 29, an end aperture 30 and a series of spirally arranged, separate side apertures 32. The main portion 24 is also provided with a series of separate, spirally arranged side apertures 34.

The inner lumen 14 has a cross sectional outer diameter substantially the same as that of the inner diameter of the distal end portion 28 of outer member 12, and is small enough to leave clearance between the side wall of outer member 12 and the side wall of inner member 14, over the length of the main portion 24 of outer lumen 12. The inner member 14 projects outwardly through arm 22 of junction piece 18, which is arranged in a straight relationship with the silastic extension 16. The inner member 14 has an enlarged end extremity 36, a male Luer lock thread 37 at its end, to receive a female Luer lock injection cap 38. End extremity 36 projects outwardly through the end of arm 22 when the inner member 14 is properly in position.

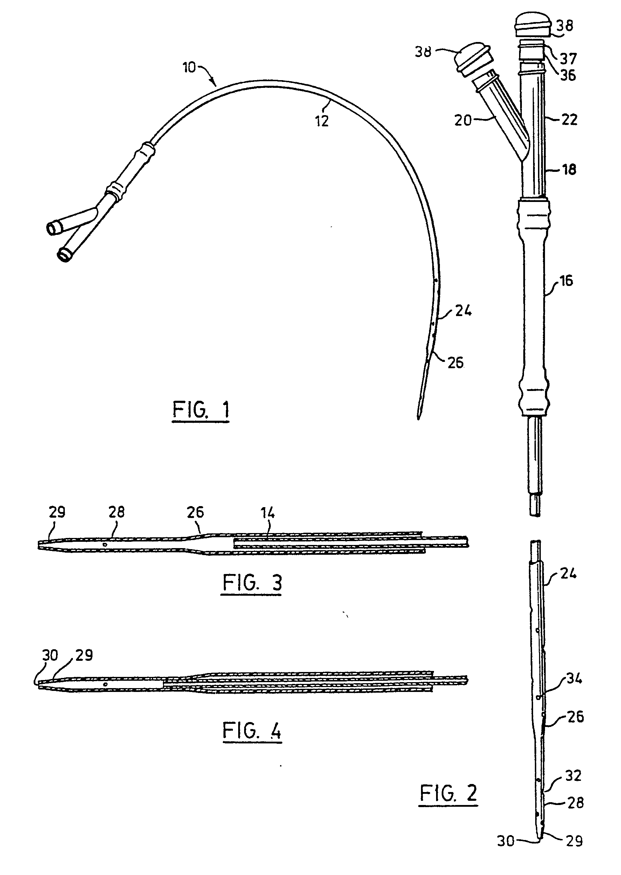

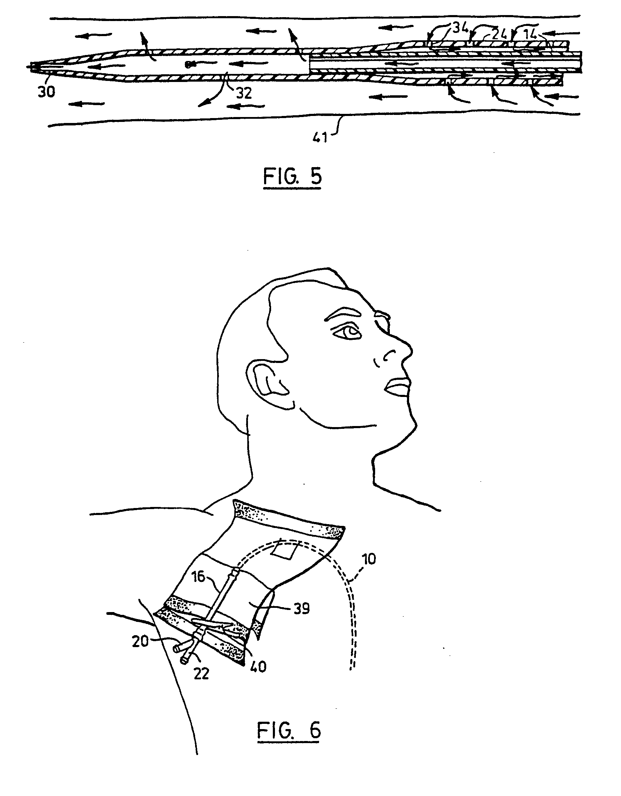

As shown in Figs. 3 and 4, the inner member 14 is withdrawable from within outer member 12, and can in fact be withdrawn all the way through extension 16 and junction piece 18. When it is inserted into the outer member 14, as shown in Fig. 4, it is pushed forwardly through inner member 14 until its end comes into engagement with convergent section 26 of outer member 12. At this position, the inner member 14 seals against the inner walls of outer member 12, and the inner member forms, with the distal end portion 28 of the outer member, a continuous inner lumen isolated from and fluid tight sealed from the outer lumen formed between the inner wall of outer member 12 and the outer wall of inner member 14. End aperture 30 and side apertures 32 in distal end portion 28 now constitute apertures for the inner lumen of the cannula. Side apertures 34 of the main portion of the outer member 12 now form apertures in the outer lumen of the cannula. When conducting a hemodialysis operation using the cannula of the present invention, blood return flow to the subclavian vein is through the inner lumen 14 and apertures 30, 32, and simultaneously blood withdrawal from the vein for dialysis treatment is through apertures 26 and outer lumen 12, and hence through arm 20 of the junction piece 18 to the machine. As shown in Fig. 5, the outer and inner lumens constitute separate, clear blood flow passageways when assembled together. The direction of blood flow, with the cannula disposed within a vein 41 is as indicated on Fig. 5. In this arrangement, treated blood is returned downstream in the vein from the location of blood withdrawal, so as to exclude the possibility of mixing within the vein of treated and untreated blood.

Fig. 6 shows the subclavian hemodialysis cannula of the present invention in position on the patient, when not in use for dialysis. The cannula is secured in position by means of adhesive dressings 9, with the rubber extension thereof 16 protruding through the dressings. A clamp 40 closes the rubber extension, when not in use, so as to guard against air ingress or contamination in the event that the junction arms 20, 22 becomes accidentally opened.

The use of a subclavian cannula as described herein largely solves the problem of temporary vascular access. Hemodialysis can be started within 20 minutes, even if the patient has no definitive permanent access. Moreover, this is done without jeopardizing future sites for arterio-venous fistula construction. Hemodialysis does not have to be delayed or postponed. Because shunts are not inserted, patients do not have to stay in hospital while shunt sites heal. Blood vessels are not destroyed, and unsightly scars are avoided. A subclavian cannula according to the invention can be inserted and the patient can go home on the same day. The cannula is well tolerated by patients both in hospital and at home. It does not significantly restrict mobility or activities. It causes no more than minimal discomfort during insertion. After it has been removed, the subclavicular scars are inconspicuous.

Whilst a specific preferred embodiment of the present invention has been illustrated and described herein in detail, it will be appreciated that this is by way of example only, and is not to be construed to be limiting. The scope of the present invention is limited only by the scope of the appended claims.

For example the same principle can be applied when dialysis or haemoperfusion for poisoning is conducted through the femoral vein. A longer cannula of the same basic design can be introduced into the inferior vena cava via the femoral vein. This will avoid the necessity of inserting two single lumen femoral cannulae.

**Claims**

1. A double lumen cannula suitable for insertion into a large vein such as the subclavian vein of a patient for blood removal therefrom and blood return thereto, the cannula comprising outer and inner tubular members (12 and 14) and characterised in that the outer tubular member (12) is elongated and flexible with a smooth outer surface and comprises a main elongated portion (24) of first cross-section, an integral convergent section (26), an integral distal end portion (28) adjoining the convergent section and having a second cross-section which is smaller than the first cross-section, a plurality of blood flow apertures (34) spaced apart from one another in the side wall of the main portion adjacent the convergent section (26), and at least one blood flow aperture (30, 32) in the distal end portion (28), and further characterised in that the inner tubular member (14) is in situ with the outer tubular member (12) and is elongated and flexible with a smaller cross-section than the first cross-section and is releasably and withdrawably disposed within the outer member (12) with its distal end within the outer lumen and in sealing end contact with the convergent section (26) of the outer member so as to define with the outer member (12) two concentrically arranged discrete fluid flow lumens, an inner lumen being constituted by the inner member (14) and the distal end portion (28) of the outer member (12) and an outer lumen constituted by the main portion (24) and the convergent section (26) of the outer member (12).

2. A cannula as claimed in claim 1 and characterised in that the distal end portion (28) of the outer member (12) has a plurality of blood flow apertures (30, 32), one (30) of said apertures being axially presented at the end thereof, the others (32) of said apertures being spaced apart from one another in the side wall thereof.

3. A cannula as claimed in claim 2 and characterised in that the blood flow apertures (32) in the side wall of the distal end portion (28) of the outer member (12) are arranged in a spiral formation.

4. A cannula as claimed in any one of claims 1 to 3 and characterised in that a junction piece (18) is provided at the end of the outer member (12) remote from the distal end portion (28) and in that the inner member (14) extends through a branch (22) of the junction piece (18).

5. A cannula as claimed in claim 4 and characterised in that a closable extension piece (16) is provided between the outer member (12) and the junction piece (18).

6. A cannula as claimed in claim 4 or claim 5 and characterised in that the distance from the distal end portion extremity (at 30) of the outer member (12) to the junction piece (18) lies in the range of 18 to 22 cms.

7. A cannula as claimed in any one of the preceding claims and characterised in that both the outer member (12) and the inner member (14) are of radio-opaque plastics material.

8. A cannula as claimed in claim 7 and characterised in that the outer member (12) is of radio-opaque tetrafluoroethylene polymer.

9. A cannula as claimed in any one of the preceding claims and characterised in that the combined length of convergent section (26) and the distal end portion (28) of the outer member (12) lies in the range of 3 to 4 cms.

10. A cannula as claimed in any one of the preceding claims and characterised in that the blood flow apertures (34) in the side wall of the main portion (24) of the outer member (12) are arranged in a spiral formation.

**Revendications**

1. Cathéter à double canal destiné à être inséré dans une grande veine, par exemple la veine sous-clavière, d'un patient pour lui retirer du sang et lui rendre du sang, le cathéter comprenant des éléments tubulaires externe et interne (12 et 14) et étant caractérisé en ce que l'élément tubulaire externe (12) est allongé et souple, d'une surface externe lisse, et comprend une partie principale allongée (24) d'une première section droite, une section solidaire convergente (26), une partie terminale distale solidaire (28) se réunissant à la section convergente et possédant une deuxième section droite qui est plus petite que la première section droite, plusieurs ouvertures (34) d'écoulement du sang réparties sans la paroi latérale de la partie principale au voisinage de la section convergente (26), et au moins une ouverture (30, 32) d'écoulement de sang ménagée dans la partie terminale distale (28), et étant caractérisé en outre en ce que l'élément tubulaire interne (14) est en place à l'intérieur de l'élément tubulaire externe (12), est allongé et souple, d'une section droite plus petite que la première section droite, et est placé, de manière à pouvoir en être retiré, à l'intérieur de l'élément externe (12) de manière que son extrémité distale soit à l'intérieur du canal externe et en contact d'étanchéité avec la section convergente (26) de l'élément externe, si bien qu'il est défini avec l'élément externe (12) deux canaux d'écoulement de fluide distincts concentriques, un canal interne étant constitué par l'élément interne (14) et la partie terminale distale (28) de l'élément externe (12), et un canal externe étant constitué par la partie principale (24) et la section convergente (26) de l'élément externe (12).

2. Cathéter selon la revendication 1, caractérisé en ce que la partie terminale distale (28) de l'élément externe (12) possède plusieurs ouvertures (30, 32) d'écoulement de sang, l'une (30) desdites ouvertures se présentant axialement à son extrémité, les autres (32) desdites ouvertures étant réparties dans sa paroi latérale.

3. Cathéter selon la revendication 2, caractérisé en ce que les ouvertures (32) d'écoulement de sang ménagées dans la paroi latérale de la partie terminale distale (28) de l'élément externe (12) sont disposées en spirale.

4. Cathéter selon l'une quelconque des revendications 1 à 3, caractérisé en ce qu'un élément de raccord (18) est prévu à l'extrémité de l'élément externe (12) qui est opposée à la partie terminale distale (28) et en ce que l'élément interne (14) passe au travers d'une branche (22) de l'élément de raccord (18).

5. Cathéter selon la revendication 4, caractérisé en ce qu'un prolongement (16) pouvant être fermé est prévu entre l'élément externe (12) et l'élément de raccord (18).

6. Cathéter selon la revendication 4 ou la revendication 5, caractérisé en ce que la distance de l'extrémité (en 30) de la partie terminale distale de l'élément externe (12) à l'élément de raccord (18) se trouve dans l'intervalle de 18 à 22 cm.

7. Cathéter selon l'une quelconque des revendications précédentes, caractérisé en ce que l'élément externe (12) et l'élément interne (14) sont tous deux faits d'une matière plastique opaque à la radioscopie.

8. Cathéter selon la revendication 7, caractérisé en ce que l'élément externe (12) est fait d'un polymère de tétrafluoroéthylène opaque à la radioscopie.

9. Cathéter selon l'une quelconque des revendications précédentes, caractérisé et ce que la longueur combinée de la section convergente (26) et de la partie terminale distale (28) de l'élément externe (12) se trouve dans l'intervalle de 3 à 4 cm.

10. Cathéter selon l'une quelconque des revendications précédentes, caractérisé en ce que les ouvertures (34) d'écoulement de sang ménagées dans la paroi latérale de la partie principale (24) de l'élément externe (12) sont disposées en spirale.

**Patentansprüche**

1. Doppellumenkatheter, geeignet zum Einführen in eine breite Vene, beispielsweise in die Unterschlüsselbeinvene eines Patienten zur Blutentnahme von diesem und Blutrückführung zu diesem, welcher Katheter eine äussere und eine innere Röhre (12 und 14) besitzt und dadurch gekennzeichnet ist, daß die äussere Röhre (12) langgestreckt und biegsam mit einer glatten Aussenfläche ist und besitzt einen langgestreckten Hauptteil (24) von einem ersten Querschnitt, einen angeformten konvergierenden Abschnitt (26), einen angeformten distalen Endteil (28) anschließend an den konvergierenden Abschnitt und mit einem zweiten Querschnitt der kleiner als der erste Querschnitt ist, eine Anzahl Blutfließöffnungen (34) in Abständen voneinander in der Seitenwand des Hauptteils benachbart dem konvergierenden Abschnitt (26) vorgesehen ist und mindestens eine Blutfließöffnung (30, 32) im distalen Endteil (28), und ferner dadurch gekennzeichnet, daß die innere Röhre (14) in situ innerhalb der äußeren Röhre (12) und langgestreckt ist und biegsam mit einem kleineren Querschnitt als der erste Abschnitt und ist lösbar und zurückziehbar innerhalb der äußeren Röhre (12) angeordnet mit ihrem distalen Ende innerhalb des äußeren Lumens und in dichtendem Endkontakt mit dem konvergierenden Abschnitt (26) der äußeren Röhre, um zu begrenzen mit der äußeren Röhre (12) zwei konzentrisch angeordnete gesonderte Fluidfließlumen, ein inneres Lumen durch die innere Röhre (14) und den distalen Endteil (28) der äußeren Röhre (12) gebildet wird und ein äußeres Lumen durch den Hauptteil (24) und den konvergierenden Abschnitt (26) der äußeren Röhre (12) gebildet wird.

2. Katheter nach Anspruch 1, dadurch gekennzeichnet, daß der distale Endteil (28) der äußeren Röhre (12) eine Anzahl Blutfließöffnungen (30, 32) aufweist, eine (30) dieser

**0 025 704**

Öffnungen ist an deren Ende axial gerichtet, die anderen (32) dieser Öffnungen befinden sich in der Seitenwand derselben in Abstand voneinander.

3. Katheter nach Anspruch 1, dadurch gekennzeichnet, daß die Blutfließöffnungen (32) in der Seitenwand des distalen Endteils (28) der äußeren Röhre (12) eine Spirale bildend angeordnet sind.

4. Katheter nach einem oder mehreren der Ansprüche 1 bis 3, dadurch gekennzeichnet, daß ein Verzweigungsstück (18) am Ende der äußeren Röhre (12), das dem distalen Endteil (28) abgekehrt ist, und daß die innere Röhre (14) sich durch einen Schenkel (22) des Verzweigungsstücks (18) erstreckt.

5. Katheter nach Anspruch 4, dadurch gekennzeichnet, daß ein verschließbares Verlängerungsstück (16) zwischen der äußeren Röhre (12) und dem Verzweigungsstück (18) vorgesehen ist.

6. Katheter nach Anspruch 4 oder 5, dadurch gekennzeichnet, daß der Abstand vom Ende (30) des distalen Endteils der äußeren Röhre (12) zu dem Verzweigungsstück (18) im Bereich von 18 bis 22 cm liegt.

7. Katheter nach einem oder mehreren der vorangehenden Ansprüche, dadurch gekennzeichnet, daß sowohl die äussere Röhre (12) als auch die innere Röhre (14) aus strahlenundurchlässigem Plast sind.

8. Katheter nach Anspruch 7, dadurch gekennzeichnet, daß die äußere Röhre (12) aus strahlenundurchlässigem Tetrafluoräthylenpolymer ist.

9. Katheter nach einem oder mehreren der vorangehenden Ansprüche, dadurch gekennzeichnet, daß die kombinierte Länge des konvergierenden Abschnitts (26) und des distalen Endteils (28) der äußeren Röhre (12) im Bereich von 3 bis 4 cm liegt.

10. Katheter nach einem oder mehreren der vorangehenden Ansprüche, dadurch gekennzeichnet, daß die Blutfließöffnungen (34) in der Seitenwand des Hauptteils (24) der äußeren Röhre (12) spiralig angeordnet sind.

0 025 704

FIG. 1

FIG. 3

FIG. 4

FIG. 2

1

FIG. 5

FIG. 6